# EUROPEAN PATENT APPLICATION

(11) **EP 4 636 381 A1**
(43) Date of publication of application: **22.10.2025**
(21) Application number: 24170550.8
(22) Date of filing: 16.04.2024
(51) Int. Cl.: G01N 1/14, B01D 29/15, G01N 1/10, G01N 1/40

(54) **ASSEMBLY FOR FILTERING AND STORING A SAMPLE SUCH AS ENVIRONMENTAL DNA**

(71) Applicant: ETH Zurich, 8092 Zurich (CH)
(72) Inventor: DEINER, Kristy, 8405 Winterthur (CH); MINTCHEV, Stefano, 4057 Basel (CH); OPIASA, Pascal, 4056 Basel (CH); PEREIRA, Catia, 8003 Zürich (CH)

(57) **Abstract**

A filter assembly (1) for filtering a sample from a fluid (F) comprises a filter holder (4), a filter membrane (5), and a filter housing (6). The filter holder (4) is at least partially received in the filter housing (6), and the filter membrane (5) is attached to the filter holder (4) and is configured to filter a sample from a fluid (F) flowing from an inlet port (2) to an outlet port (3) through the filter membrane (5). The filter holder (4) is removably attached to the filter housing (6) such, that the filter holder (4) together with the filter membrane (5) is removable from the filter housing (6) and is insertable into a storage assembly (100) comprising a storage housing (20).

## Description

### TECHNICAL FIELD

The present invention relates to a filter assembly according to claim 1, a storage assembly according to claim 7, a kit-of-parts comprising a filter assembly and a storage housing according to claim 9, a method of filtering a sample with such a kit-of-parts according to claim 13, a method of analyzing a sample with such a kit-of-parts according to claim 14, and to a method of manufacturing such a kit-of-parts according to claim 15.

### PRIOR ART

Anthropogenic impacts have led to a rapid decline global biodiversity, resulting in lost ecosystem function and modified and natural habitats. As primary producers and carbon sinks, plant communities are fundamental in food production, health and have a huge impact on the global climate. Thus, the importance of consistent and standardized monitoring of total animal and plant biodiversity has been recognized. Conventional monitoring methods (e.g., physical, acoustic and visual-based) to assess plant biodiversity have limitations. For example, the differences in taxonomic identification among experts or when surveying small-bodies or elusive species. In conventional plant biodiversity monitoring ephemeral, cryptic and dormant plants or plant parts are missing diagnostic characters that may lead to underestimation of plant diversity and abundance. Monitoring is indispensable for conservation strategies and effective management for target species or communities over large temporal and spatial scales.

To obtain information such as distribution or the consequences of changing biodiversity it is well-known to collect samples from the environment, for instance environmental DNA (eDNA). In fact, the use of eDNA analysis has revolutionized total biodiversity assessment and monitoring, especially invasive and rare species. eDNA can be captured directly from an environmental sample (e.g. air, soil, water or ice) without isolating the target organisms High-throughput sequencing is used to target a specific DNA sequence called DNA barcode. These sequences can then be compared to a sequence database to assign the scientific name of a species to which the DNA may belong. Biodiversity assessment and monitoring using eDNA metabarcoding has been conducted in several environments. However, the environment from which eDNA is sampled and analyzed from directly affects the quality and quantity of eDNA that will be detected.

eDNA degradation and transport play a key role in the interpretation of molecular data generated from molecular analyses. Degradation of eDNA depends on abiotic (temperature, UV, pH, ions) and biotic (microbial activity) factors.

Most eDNA studies in aquatic environments utilize some form of filtration to capture eDNA, however there is trade-off between the volume of water that can be pushed through and a mesh size of the filter membrane. In most aquatic systems, fine mesh size filter membranes tend to clog. Conversely, using large mesh size and membrane area can facilitate filtration, however at a cost of losing out on finer fractions of eDNA.

Furter disadvantages of the known methods for collecting samples based on filtering are filter assemblies that require to break open a filter housing to have access to the filter membrane causing significant handling time or perform the laboratory steps inside the filter housing which causes significant loss in sample yield.

From US 2023/0211294 A1 a self-preserving filter is known, but this only works with a vacuum pump and does not take into account any post processing steps after storage. Further disadvantages of filter assemblies used in the field for large volumes required expensive pumps and batteries powering them, leading to localized use to where this equipment can be carried and function, e.g., battery life in cold conditions is significantly impaired.

Furthermore, none of the known prior art solutions address two main issues of the preservation of the sample in the field in combination with a transition of the filtered sample from the filter assembly to an analysis assembly such as a centrifuge tube needed for subsequent processing and analysis steps.

### SUMMARY OF THE INVENTION

It is an object of the present invention to overcome the drawbacks of the prior art. In particular, it is an object to provide a filter assembly that enables improved transferring and handling of filter membranes.

This object is achieved with a filter assembly according to claim 1. In particular, a filter assembly for filtering a sample from a fluid is provided, wherein the filter assembly comprises an inlet port, an outlet port, a filter holder, a filter membrane, and a filter housing. The filter holder is at least partially received in the filter housing. The filter membrane is attached to the filter holder and is configured to filter a sample from a fluid flowing from the inlet port to the outlet port through the filter membrane. The filter holder is removably attached to the filter housing such, that the filter holder together with the filter membrane is removable from the filter housing.

The sample preferably is environmental DNA (eDNA) that is filtered from an environmental fluid such as from an environmental liquid, for instance environmental water, or from an environmental gas. In particular, the filter assembly is configured to filter a sample from water in any of its forms, i.e. solid, liquid and gasous. The liquid forms are many, such as rain, wastewater, tap water, deionized water, molecular grade water, recirculating aquarium, aquiculture or swimming pool water. Examples of solid forms are ice, freezing rain, hail, sleet and snow. In this case it is preferred that the solid form of water is first melted to liquid form and then filtered with the filter assembly. A conceivable gaseous form is water vapor. To this end it is preferred that water vapor is collected, condensed to liquid, and then filtered with the filter assembly. Another example of eDNA sources is clouds that are an interesting state as they are a mix of water vapor and liquid state. But this too could be sampled by condensation.

The environmental DNA preferably comprises or consist of material stemming from dead and/or from living organisms and that preferably comprises single-stranded (ss) and/or double-stranded (ds) DNA fragments from nuclear and/or mitochondrial and/or plastid DNA of eukaryotes and/or plasmid DNA of prokaryotes. For instance, eDNA can comprise or consist of cellular DNA from gametes, tissues, feces or extracellular DNA originating from cells and organelles or particle bound DNA such as that to clay or sand.

The filter assembly is preferably configured to adapt a filtering state, wherein the filter holder is at least partially received in the filter housing and the filter membrane is attached to the filter holder to filter a sample from the fluid.

As will be explained in greater detail further below, the filter holder and the filter membrane can also be configured for a storage state, wherein the filter holder and the filter membrane are at least partially insertable into a storage housing.

Hence, the filter holder and the filter membrane are preferably configured for filtering a sample and for storing the filtered sample. As such, the filter holder and the filter membrane can be said to have a dual function.

As will be explained in greater detail below, the filter assembly is preferably configured to meet the European standard CEN EN 17805:2023 ("Water quality - Sampling, capture and preservation of environmental DNA from water", approved by CEN on 30 January 2023) and/or to filter a sample from a fluid in accordance with said European standard.

The filter holder together with the filter membrane is preferably indestructibly removable from the filter housing. In other words, the filter holder together with the filter membrane preferably remains intact when the filter holder is removed from the filter housing.

The filter holder and/or the filter housing preferably are UV-resistant and/or bleach resistant so that they can be decontaminated and, consequently, re-used for filtering further samples.

The filter holder and/or the filter membrane and/or the filter housing preferably have an elongated shape, for instance a cylindrical shape.

Preferably, the filter housing, the filter membrane and the filter holder are arranged concentrically. In particular, in a connected state of the filter holder to the filter housing and when seen along a radial direction extending from a central axis running centrally through the filter holder radially outwards, the filter membrane is arranged after the filter holder and the filter housing is arranged after the filter membrane. In other words, when the filter assembly is seen in cross-section, it is preferred that the filter membrane is arranged between the filter housing and the filter holder.

Furthermore, in the connected state, the filter holder is preferably partially arranged in the filter housing and the filter membrane is preferably completely arranged in the filter housing.

The filter holder is preferably arranged in the filter housing in such a way that fluid flowing from the inlet port to the outlet port flows laterally through the filter membrane and through the filter holder into the filter holder, in particular along the radial direction. It is furthermore preferred that the fluid flows along a longitudinal direction of the filter holder.

The filter holder preferably has through-openings through which the fluid can flow and from outside of the filter holder into the filter holder, in particular along the radial direction. The through-openings are preferably elongated and extend at least partially along the longitudinal direction of the filter holder.

Preferably, the filter holder has an interior space. The interior space is preferably limited or confined by a jacket wall of the filter holder that has the through-openings. The jacket wall preferably comprises the aforementioned outer surface on which the filter membrane is arranged.

The filter membrane is preferably attached to an outer surface of the filter holder and preferably extends at least partially around the outer surface of the filter holder.

The filter membrane is preferably removably attached to the filter holder, preferably via a frictional fit.

The filter membrane preferably is hydrophilic. The filter membrane preferably comprises or consists of one or more hydrophilic polymers such as a thermoplastics, for instance a synthetic polymer such as nylon.

Additionally or alternatively, the filter membrane preferably comprises or consists of a material that has thermal and pressure stability up to 1.5 bar at 85 °C and/or high flow rates greater than 20 mL/min/cm2 @ 1 bar and/or sterilizability through methods like gamma irradiation and/or maintain integrity in range of pH that may have varying levels of acidity or alkalinity preferably in the range of 5 - 13.

A thickness of the filter membrane preferably is in the range of 25 micrometer to 400 micrometer, such as in the range of 50 micrometer to 200 micrometer, more preferably in the range of 80 micrometer to 120 micrometer. Additionally or alternatively, a thickness of the filter membrane preferably is 400 micrometer or less.

The filter membrane preferably comprises a mesh having mesh openings, for instance of a size of 0.1 micrometer or more, preferably of 0.22 micrometer or more.

The filter housing and the filter holder in each case preferably have at least one connection element that can be detachably connected to each other, the connection elements preferably being threaded elements. Additionally or alternatively, the filter holder is preferably rotatably mounted to the filter housing and connectable to the filter housing via rotation about an axis of rotation.

That is, the filter housing preferably has at least one connection element and the filter holder preferably has at least one connection element which can be releasably connected to each other, preferably detachably engaged with one another. The connection elements are preferably threaded elements. To this end it is preferred that the filter housing has an external thread and that the filter holder has an internal thread.

The filter holder can thus be rotated relative to the filter housing, in particular the filter holder can be connected to the filter housing by rotation about an axis of rotation running centrally through the filter housing. The axis of rotation preferably runs parallel to a central axis of the filter housing and in particular also parallel to the central axis of the filter holder and/or the filter membrane.

The filter holder preferably comprises a lid and the filter housing preferably comprises an opening, wherein the filter holder is insertable into the filter housing via the opening of the filter housing, and wherein the lid of the filter holder is configured to at least partially close the opening of the filter housing when the filter holder is received in the filter housing.

The filter holder, in particular the lid, preferably comprises the outlet port. Additionally or alternatively, the filter housing preferably comprises the inlet port. That is, the inlet port is preferably provided on the filter housing. The outlet port is preferably provided on the filter holder, particularly preferably on the lid of the filter holder.

As mentioned earlier, the filter holder preferably has a connection element for connection with a connection element of the filter housing. It is preferred that the connection element of the filter holder is provided on the lid of the filter holder. It is furthermore preferred that the connection element of the filter housing is provided in a region of the opening of the filter housing. In particular, the lid preferably has an internal thread arranged along an inner circumference of the lid, which can engage with an external thread running along an outer circumference of the filter housing.

The outlet port preferably extends completely through the lid and away from the lid towards an outside of the filter assembly.

The inlet port preferably extends away from the filter housing and towards an outside of the filter assembly.

It is further preferred that the inlet port and the outlet port are arranged opposite to each other with respect to a central axis of the filter assembly. The central axis of the filter assembly preferably runs parallel to the central axis of the filter housing and of the filter holder and of the filter membrane, respectively.

The filter assembly preferably comprises at least one sealing element, particularly preferably a sealing cap, that is configured to seal the outlet port and/or the inlet port. Additionally or alternatively, the sealing element and the outlet port and/or the inlet port in each case preferably have at least one connection element that can enter a preferably releasable connection with one another.

That is, the filter assembly preferably comprises at least one sealing element such as a sealing cap that can seal the outlet port and/or the inlet port. To this end it is preferred that the sealing element has at least one connection element such as a threaded element and that the outlet port and/or the inlet port has at least one corresponding connection element such as a threaded element, such that the sealing element can be preferably releasably connected to the outlet port and/or the inlet port. For instance, the sealing element can comprise an internal thread and the outlet port (inlet port) can comprise an external thread, such that the sealing element can be screwed onto the outlet port (inlet port) and thereby seal the outlet port (inlet port) and thus the filter assembly towards an outside. That is, the sealing element preferably is a threaded sealing cap.

The outlet port is preferably connectable to a pumping device being configured to generate a suction force in the filter assembly that sucks the fluid into the filter assembly through the inlet port and out of the filter assembly through the outlet port.

The pumping device particularly preferably is a hand-operated pumping device such as a transfer pump, for instance as used on boats for pumping out bilge water or for syphoning gas out of a car. These pumping devices are inexpensive and widely available. That is, the pumping device preferably is a commercially available pumping device. Since the pumping device preferably is hand-operated, a power source such as a battery can be dispensed with.

The filter assembly preferably further comprises at least one adapter device that is connectable to the outlet port and further connectable to the pumping device, preferably via at least one pumping hose. That is, the outlet port is preferably connected to the pumping device via at least one adapter device. The adapter device preferably serves the purpose of enabling a connection of the filter assembly, in particular of the outlet port, with a pumping device or other component that is of a different size and/or shape. For instance, the outlet port is preferably in connection with the pumping device via at least one pumping hose. The adapter device is preferably configured and/or serves the purpose of allowing a connection in the event that a nominal pipe size of the pumping hose and of the outlet port is different.

The adapter device preferably has at least one connection element configured for connection with the connection element of the outlet port. In particular, the adapter device preferably comprises a thread such as an internal thread and is configured to be screwed onto the external thread of the outlet port. Furthermore, the adapter device preferably comprises an outlet port for connection to the pumping hose, whereby the pumping hose can in turn be connected to the pumping device. In addition, the adapter device preferably comprises a through-opening via which the fluid can be sucked from the outlet port of the filter holder through the adapter device and out of the outlet port of the adapter device and, for instance, via the pumping hose to the pumping device.

Preferably, the pumping hose can be connected to the outlet port of the adapter device by means of a frictional connection. In particular, the pumping hose can be plugged on the outlet port of the adapter device. However, other connections are just as conceivable, for example the pumping hose could be screwed to the outlet port.

The filter assembly is preferably configured to filter the fluid with a filtration rate of 5 liter per minute or more, achievable for example with an inlet port greater than 1.0 cm and high surface area of the filter membrane, preferably more than 35 cm².

In another aspect, a storage assembly is provided. The storage assembly comprises at least one storage housing, and at least one filter holder and at least one filter membrane as described above. The filter holder together with the filter membrane can be stored in the storage housing.

Any statements the filter assembly, in particular the filter holder and the filter membrane, preferably likewise apply to the storage assembly and vice versa.

The storage housing preferably serves the purpose of storing the filter holder together with the filter membrane after a filtering of a sample with the filter assembly has been performed. That is, the storage housing is preferably configured to store the filter holder and the filter membrane being attached to the filter holder after the filter holder together with the filter membrane are removed from the filter housing. To this end it is preferred that the filter holder and the filter membrane are at least partially insertable into the storage housing. This inserted state preferably forms the storage state mentioned initially.

The storage housing preferably has an elongated shape, preferably a cylindrical shape. In particular, the storage housing is preferably in the form of a storage tube.

In the storage state it is preferred that the storage housing, the filter membrane and the filter holder are arranged concentrically. In particular, in the storage state and when seen along a radial direction extending from the central axis running centrally through the filter holder radially outwards, the filter membrane is arranged after the filter holder and the storage housing is arranged after the filter membrane. In other words, when seen in cross-section, it is preferred that the filter membrane is arranged between the storage housing and the filter holder.

Furthermore, in the storage state, the filter holder is preferably partially arranged in the storage housing and the filter membrane is preferably completely arranged in the storage housing.

Preferably, the storage housing has a similar design to the filter housing. In particular, the storage housing is preferably configured as the filter housing, with the difference that the storage housing lacks the inlet port.

Thus, the storage housing preferably comprises an opening, wherein the filter holder is insertable into the storage housing via the opening of the storage housing.

The storage housing is preferably a commercially available storage tube, such as a centrifuge tube.

The storage housing preferably has at least one connection element that is releasably connectable to the connection element of the filter holder. The connection element of the storage housing preferably is a threaded connection element. To this end it is preferred that the storage housing has an external thread that can engage the internal thread of the filter holder.

The filter holder is thus preferably rotatable relative to the storage housing. In particular, the filter holder is preferably connectable to the storage housing by rotation about an axis of rotation running centrally through the storage housing. The axis of rotation preferably runs parallel to a central axis of the storage housing and in particular also parallel to the central axis of the filter holder and/or the filter membrane.

As mentioned earlier, the filter holder can comprise a lid, and wherein the connection element of the filter holder, in particular the internal thread, is preferably provided in the lid. Hence, upon insertion of the filter holder into the storage housing the lid of the filter holder is preferably configured to at least partially close the opening of the storage housing.

The outlet port is preferably provided on the filter holder and the storage assembly preferably comprises at least one sealing element, preferably a sealing cap, that is configured to seal the outlet port. The sealing element preferably corresponds to a sealing element as mentioned earlier. That is, the sealing element preferably has at least one connection element such as a threaded element, for instance an internal thread, such that the sealing element can be screwed onto the outlet port and thereby seal the outlet port and thus the storage assembly towards an outside.

Said sealing element preferably corresponds to the aforementioned sealing element, i.e. the sealing element for closing the outlet port of the filter assembly preferably is the sealing element for closing the outlet port of the storage assembly and vice versa.

That is, the filter assembly comprising the filter holder and the filter membrane at least partially received in the filter housing is preferably used to filter a sample. After filtering, the outlet port can be closed with the sealing element and the filter holder together with the filter membrane and the closed outlet port can be transferred into the storage housing. However, it is likewise conceivable that after filtering, the filter holder together with the filter membrane is first transferred into the storage housing, and wherein the outlet port of the filter holder is closed by the sealing element once the filter holder and the filter membrane are received in the storage housing.

In another aspect, a kit-of-parts for filtering a sample from a fluid and for storing the filtered sample is provided. The kit-of-parts comprises at least one filter assembly as described above, and at least one storage housing as described above. The filter holder together with the filter membrane can be stored in the storage housing. The filter holder together with the filter membrane being stored in the storage housing preferably provides the storage assembly mentioned earlier.

Any statements regarding the filter assembly and the storage assembly preferably likewise apply to the kit-of-parts and vice versa.

The kit-of-parts preferably comprises instructions comprising the steps of i) filtering the sample from the fluid with the filter assembly 1, ii) removing the filter holder together with the filter membrane from the filter housing of the filter assembly, and iii) transferring the filter holder together with the filter membrane into the storage housing.

Steps i) to iii) are preferably carried out in this order and consecutively.

Step i) of the instructions preferably specify the filtering in accordance with the European standard CEN EN 17805:2023 ("Water quality - Sampling, capture and preservation of environmental DNA from water", approved by CEN on 30 January 2023) mentioned earlier

The storage housing is prefilled with a preservation solution that is configured to preserve a sample being filtered with the filter assembly.

Various preservation solutions are conceivable. For instance, the preservation solution preferably is an aqueous solution, for example a saline solution. Another example is a preservation solution in the form of a buffer solution that comprises at least one buffer. For example, the preservation solution could be a solution comprising ethylenediaminetetraacetic acid (EDTA), tris(hydroxymethyl)aminomethane hydrochloride (Tris-HCl), and sodium chloride (NaCl). For instance, the preservation solution could comprise or consist of 0.5M EDTA, 1M Tris-HCl, and 5M NaCl.

The preservation solution preferably depends on the sample being filtered. For instance, if the sample being filtered is eDNA, a preferred preservation solution preferably is a buffer solution as just described.

In case of a prefilled storage housing, it is preferred that the storage housing is closed with a removable closure element. For example, the storage housing can be a prefilled storage tube, the opening of which is closed with a closure element such as a screw cap that needs to be removed from the opening of the storage housing before the filter holder and filter membrane can be inserted into the storage housing and its preservation solution.

The kit-of-parts preferably comprises at least one pumping device that is connectable to the outlet port and is configured to generate a suction force in the filter assembly that sucks the fluid into the filter assembly through the inlet port and out of the filter assembly through the outlet port, the pumping device preferably being a hand-operated pumping device.

Additionally or alternatively, the kit-of-parts preferably comprises at least one adapter device that is connectable to the outlet port and further connectable to a pumping device, preferably via at least one pumping hose.

The pumping device preferably corresponds to the pumping device described earlier. The adapter device preferably corresponds to the adapter device described earlier.

The instructions preferably further comprise the step of connecting the pumping device to the outlet port, preferably via an adapter device, and operating the pumping device in order to generate a suction force in the filter assembly that sucks the fluid into the filter assembly.

The instructions preferably further comprise the step of connecting the adapter device to the outlet port and to the pumping device, preferably via at least one pumping hose that is attached to the adapter device and the pumping device.

The kit-of-parts can comprise further components such as the pumping hose.

Additionally or alternatively the kit-of-parts can comprise at least one inlet hose that is connectable to the inlet port, and wherein the fluid is sucked into the inlet port via the inlet hose. The instructions preferably further comprise the step of connecting the inlet hose to the inlet port.

Additionally or alternatively the kit-of-parts can comprise at least one analysis assembly that is configured to at least partially receive the filter membrane and to be analysed with an analysis device being configured to analyse the filtered sample.

The analysis assembly preferably comprises at least one analysis housing that is configured to receive, at least partially and preferably entirely, the filter membrane. The analysis assembly preferably further comprises at least one closure element that is configured to removably close an opening of the analysis housing. For example, the analysis housing can be a tube, preferably a commercially available tube such as a centrifuge tube, and the opening of which can be closed with a closure element such as a screw cap.

The analysis assembly can be analysed with an analysis device that is configured to perform an extraction analysis in order to analyse the filtered sample on the filter membrane while said filter membrane is at least partially received in the analysis assembly. This means that the filter holder and/or other components of the filter assembly and/or of the storage assembly are preferably not added to the analysis assembly and/or subjected to the analysis device. Instead, it is preferred that only the filter membrane is transferred into the analysis assembly when analyzed with the analysis device for extraction of for example DNA, optionally while being arranged in the analysis assembly or parts thereof for purification of DNA through methods of membrane lysis consisting of mechanical, chemical or temperature or any combination thereof. Through use of a single tube for storage and lysis, this results in a higher sample yield and consequently improved sensitivity.

The instructions preferably further comprise the steps of removing the filter holder together with the filter membrane from the storage housing, separating the filter membrane from the filter holder, and transferring the filter membrane into the analysis housing.

The components of the kit-of-parts are preferably provided in a bucket or the like.

An exemplary kit-of-parts may comprise the following components:
- a bucket, for example a plastic bucket,
- a pumping device, in particular a hand-operated pumping device,
- a filter holder with an attached filter membrane,
- a prefilled storage housing,
- an analysis assembly,
- a pumping hose, and
- an inlet hose.

The kit-of-parts can further comprise utensils as commonly used for filtration and storage, e.g. gloves for the user of the filter assembly and/or the storage assembly or the user of the kit-of-parts, or a bag or the like for storing the storage assembly after use, i.e. for storing the storage housing comprising the filter holder together with the filter membrane after the sample has been filtered.

In another aspect, a method of filtering a sample from a fluid and of storing the filtered sample is provided. Said method comprises the steps of i) providing a kit-of-parts as described above, ii) filtering the sample from the fluid with the filter assembly iii) removing the filter holder together with the filter membrane from the filter housing of the filter assembly, and iv) transferring the filter holder together with the filter membrane into the storage housing.

Steps i) to iv) are preferably carried out in this order and consecutively.

Any statements regarding the filter assembly, the storage assembly, and the kit-of-parts preferably likewise apply to the method of filtering the sample from the fluid and storing the filtered sample and vice versa.

For instance, the filtering in step ii) is preferably carried out in accordance with the European standard CEN EN 17805:2023 ("Water quality - Sampling, capture and preservation of environmental DNA from water", approved by CEN on 30 January 2023) and/or in accordance with step i) of the instructions of the kit-of-parts mentioned earlier.

The sample preferably is environmental DNA and/or the fluid preferably is an environmental fluid, in particular an environmental liquid such as environmental water or an environmental gas as mentioned initially.

In another aspect, a method of analyzing a sample from a fluid is provided. Said method comprises the steps of i) filtering a sample from a fluid and storing the filtered sample with a kit-of-parts as described above and/or according to the method of filtering a sample from a fluid and of storing the filtered sample as described above, ii) removing the filter holder together with the filter membrane from the storage housing, and iii) analyzing the filter membrane comprising the filtered sample preferably after the filter membrane has been removed from the filter holder and optionally has been transferred into the analysis assembly.

Steps i) to iii) are preferably carried out in this order and consecutively.

Any statements regarding the filter assembly, the storage assembly, the kit-of-parts, and the method of filtering the sample from the fluid and storing the filtered sample preferably likewise apply to the method of analyzing the sample and vice versa.

For instance, the filtering in step i) is preferably carried out in accordance with the European standard CEN EN 17805:2023 ("Water quality - Sampling, capture and preservation of environmental DNA from water", approved by CEN on 30 January 2023) and/or in accordance with step i) of the instructions of the kit-of-parts mentioned earlier.

The sample preferably is environmental DNA and/or the fluid preferably is an environmental fluid, in particular an environmental liquid such as environmental water or an environmental gas as mentioned initially.

That is, it is preferred that only the filter membrane comprising the filtered sample is used for the analysis of the filtered sample. In other words, the filter membrane is preferably removed from the filter holder and subsequently transferred into the analysis assembly and/or analyzed with an analysis device, for instance.

In another aspect, a method of manufacturing a kit-of-parts is provided. Said method comprises the steps of i) providing at least one filter assembly as described above, and ii) providing at least one storage housing as described above.

Any statements regarding the filter assembly, the storage assembly, the kit-of-parts, the method of filtering the sample from the fluid and storing the filtered sample, and the method of analyzing the sample preferably likewise apply to the method of manufacturing the kit-of-parts and vice versa.

The filter holder and/or the filter housing and/or the storage housing and/or the analysis housing are preferably provided by 3D-printing or molding.

As mentioned initially, the filter holder and/or the filter housing preferably are UV-resistant and/or bleach resistant so that they can be decontaminated and re-used for filtering further samples.

Likewise, the storage housing and/or the analysis housing preferably are UV-resistant and/or bleach resistant and/or re-usable for storing further samples. In fact, further components of the filter assembly, the storage assembly and the analysis assembly such as the sealing element of the filter holder, in particular the lid, the adapter device, the closure element of the storage housing or the closure element of the analysis housing are preferably UV-resistant and/or bleach resistant and/or re-usable.

For instance, at least one of the filter holder, the filter housing, the storage housing and further components of the filter assembly and the storage assembly such as the sealing element of the filter holder, in particular the lid, the adapter device or the closure element of the storage housing preferably comprise or consist of a thermoplastic such as ASA filament (acrylonitrile styrene acrylate) when injection molding is used or Nylon PA12 (Plastic) when 3D printing is used.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a schematical view of a method of filtering a sample from a fluid with a filter assembly being connected to a pumping device according to the invention;
- Fig. 2: shows a sectional view of section A of figure 1;
- Fig. 3: shows an exploded view of the filter assembly of figure 1,;
- Fig. 4: shows a perspective view of the filter assembly of figure 1;
- Fig. 5: shows a perspective view of a storage assembly according to the invention, wherein a filter holder and a filter membrane of the filter assembly are inserted into a storage housing of the storage assembly;

- Fig. 6: shows a perspective view of the filter assembly of figure 5;
- Figs. 7a: shows a perspective view of the filter holder together with the filter membrane being removed from the storage housing;
- Fig. 7b: shows a perspective view of the filter membrane being removed from the filter holder and being arranged in an analysis housing of an analysis assembly according to the invention;
- Figs. 7c -7b: show perspective views of the filter membrane being inserted into the analysis housing of the analysis assembly, and wherein the analysis housing is closed by a closure element of the analysis assembly.

### DESCRIPTION OF PREFERRED EMBODIMENTS

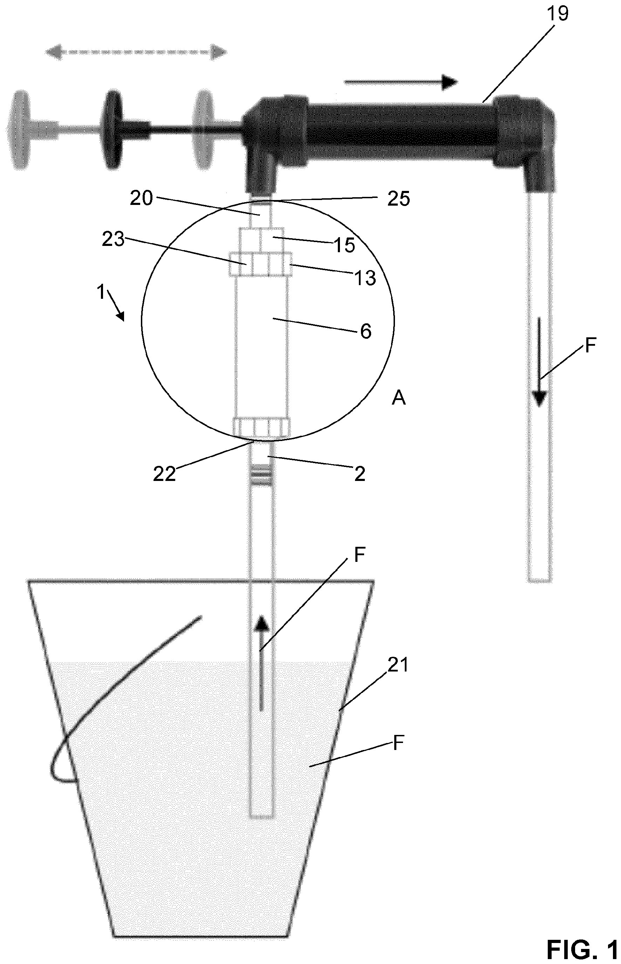

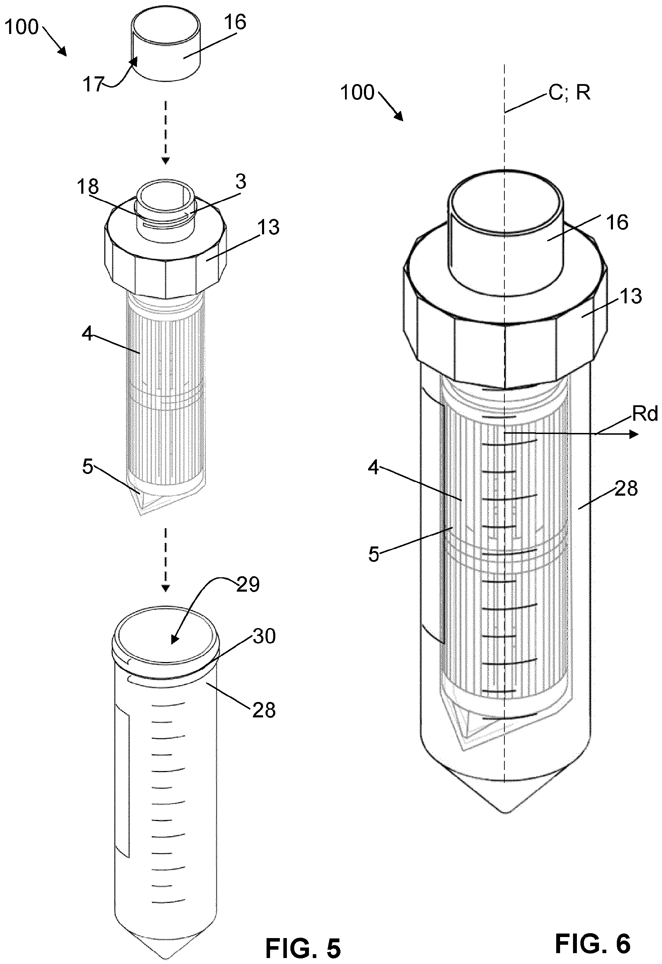

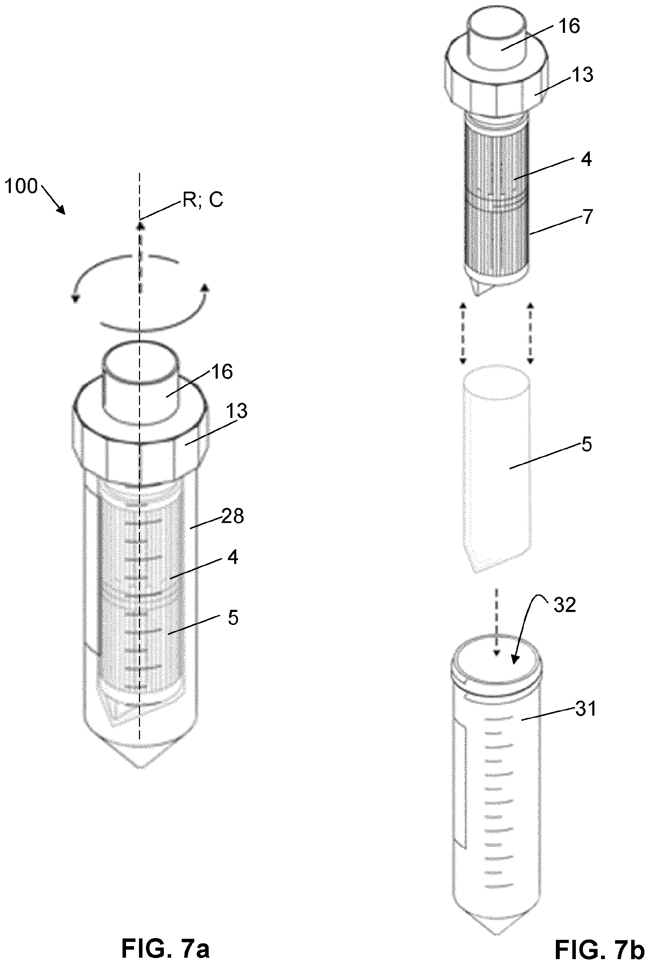

With reference to the figures, aspects of the invention will now be illustrated. In particular, the present invention solves the challenges of filtering a sample from a fluid F with a filter assembly 1 in the field at high-volume sampling with an inexpensive and easily available hand-operated pumping device 19, see figures 1 to 4, storing the filtered sample in a storage assembly 100, see figures 5 to 6, and analyzing the filtered sample in an analysis assembly 1000, see figures 7a to 7d. That is, the assemblies 1, 100, 1000 can be seen as constituting a filtering, storing and analyzing system that is designed for the filtering and preservation in the field and later analysis of a sample such as environmental DNA.

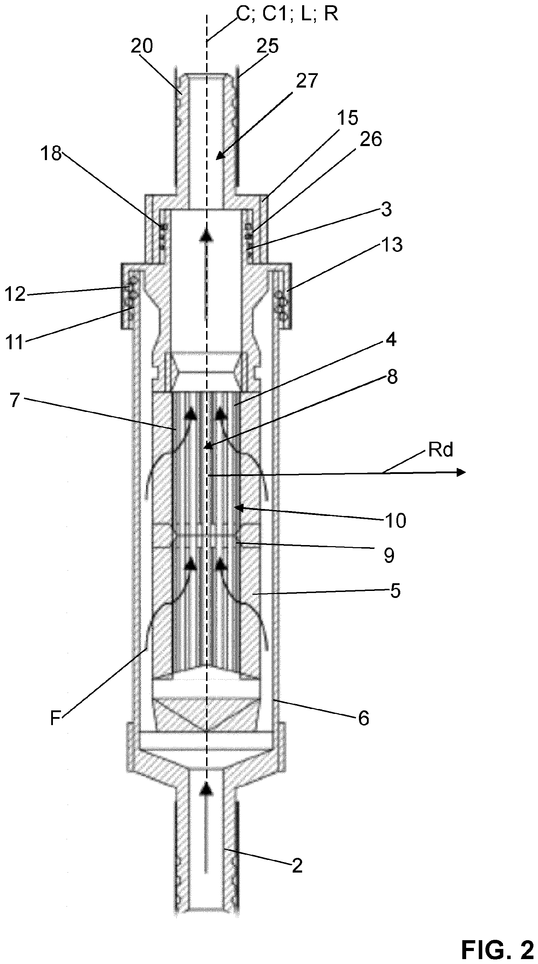

As follows from figures 1 to 4, the filter assembly 1 comprises an inlet port 2, an outlet port 3, a filter holder 4, a filter membrane 5, and a filter housing 6. In a connected state as shown in figures 1, 2 and 4, the filter holder 4 is partially arranged in the filter housing 6 and the filter membrane 5 is completely arranged in the filter housing 6. In this connected state, the filter housing 6, the filter membrane 5 and the filter holder 4 are arranged concentrically, see figure 2. That is, when seen along a radial direction Rd of the filter holder 4 extending from a central axis C of the filter holder 4 running centrally through the filter holder 4 radially outwards, the filter membrane 5 is arranged after the filter holder 4 and the filter housing 6 is arranged after the filter membrane 5. The filter holder 4, the filter membrane 5 and the filter housing 6 each have an elongated, cylindrical shape. Furthermore, the filter holder 4 comprises a lid 13 and the filter housing 6 comprises an opening 14, wherein the filter holder 4 is insertable into the filter housing 6 via the opening 14 of the filter housing 6. The lid 13 of the filter holder 4 partially closes the opening 14 of the filter housing 6 when the filter holder 4 is received in the filter housing 6. In fact, the lid 13 is configured to extend over the opening 14 of the filter housing 6 but comprises the outlet port 3. Due to the presence of the outlet port 3 the lid 13 is said to be partially closing the outlet opening 14. The filter housing 6 comprises the inlet port 2. As can also be seen from these figures, the outlet port 3 and the inlet port 2 are opposite each other when viewed along the central axis C of the filter holder 4 that runs parallel to a central axis C1 of the filter assembly 1. In other words, the inlet port 2 is arranged on a lower side 22 of the filter assembly 1 facing the source of the fluid F, that corresponds to a bucket 21 of water in figure 1 for illustrative purposes, and the outlet port 3 is arranged on an upper side 23 of the filter assembly 1 facing away from the fluid F and opposite the lower side 22. The outlet port 3 extends completely through the lid 13 and away from the lid 13 towards an outside of the filter assembly 1. The inlet port 2 as well extends away from the filter housing 6 and towards the outside of the filter assembly 1, although along an opposite direction.

As follows from figures 1 to 4, the outlet port 3 is connectable to the pumping device 19. Upon operation, the pumping device 19 generates a suction force in the filter assembly 1 that sucks the fluid F into the filter assembly 1 through the inlet port 2 and out of the filter assembly 1 through the outlet port 3. The dashed arrow in figure 1 indicates a pumping movement and the solid arrows indicate a flow direction of the fluid F during operation of the pumping device 19.

The filter holder 4 is arranged in the filter housing 6 in such a way that fluid F flowing from the inlet port 2 to the outlet port 3 flows along a longitudinal direction L of the filter holder 4 and laterally through the filter membrane 5 and through the filter holder 4 into an interior space 8 of filter holder 4 along the radial direction, see figure 2. The interior space 8 is limited or confined by a jacket wall 9 of the filter holder 4 that has through-openings 10 through which the fluid F can enter from an outside of the filter holder 4 into the interior space 8 of the filter holder 4. The through-openings 10 are of an elongated shape and extend along the entire jacket wall 9 of the filter holder 4 when seen along the longitudinal direction L of the filter holder 4. The lid 13 and the jacket wall 9 are a single-piece element. The jacket wall 9 comprises an outer surface 7 on which the filter membrane 5 is removably arranged. In fact, the filter membrane 5 is removably attached to the filter holder 4 via a frictional fit that is established between an inner surface 24 of the filter membrane 5 and the outer surface 7 of the filter holder 4 when the filter membrane 5 is placed over the filter holder 4, in particular over the jacket wall 9. An inner diameter of the filter membrane 5 thus essentially corresponds to an outer diameter of the filter holder 4, in particular of the jacket wall 9. To separate the filter membrane 5 from the filter holder 4, the filter membrane 5 can simply be pulled away from the filter holder 4, releasing the friction fit. The filter membrane 5 is formed of a mesh consisting of a hydrophilic material, for instance nylon, that has mesh openings through which the fluid F can flow through the filter membrane 5 into the filter holder 4, whereby the filter membrane 5 filters the sample from the fluid F.

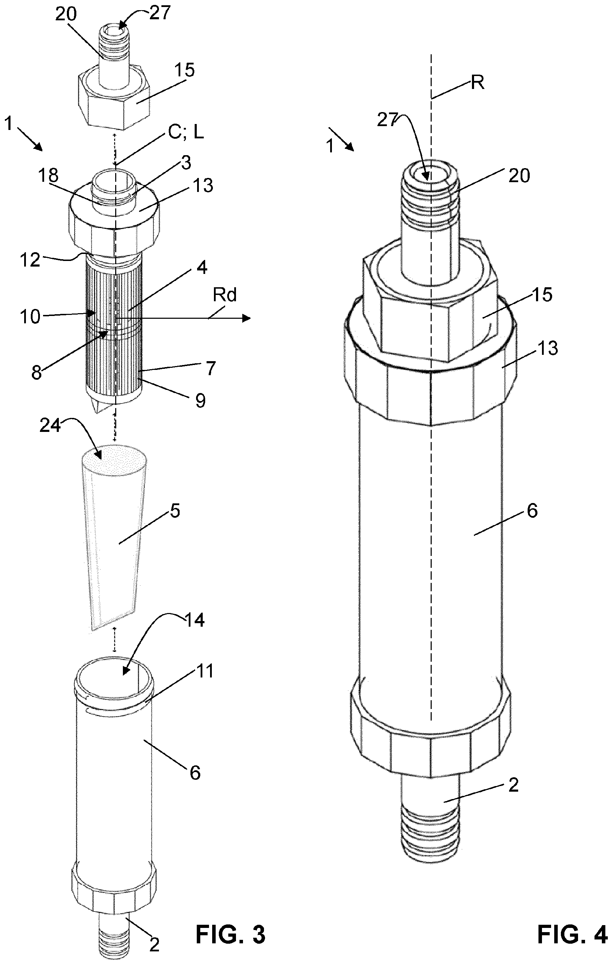

As best visible in figure 3, the filter assembly 1 comprises an adapter device 15 that is connectable to the outlet port 3 and further connectable to the pumping device 19, for instance via at least one pumping hose 25 as depicted in figure 1.

The adapter device 15 preferably has a connection element 26 in the form of an internal thread that can be connected to a connection element 18 in the form of an external thread of the outlet port 3, see figures 2 and 3.

In addition, the adapter device 15 comprises a through-opening 27 that is in fluid F connection with the outlet port 3 of the filter holder 4 and via which the fluid F can be sucked from the outlet port 3 of the filter holder 4 through the adapter device 15 and out of the outlet port 3 of the adapter device 15 and, for instance, via the pumping hose 25 to the pumping device 19. In particular, when the adapter device 15 is connected to the filter holder 4, the outlet port 3 of the filter holder 4 opens into the through-opening 27 of the adapter device 15.

Furthermore, the adapter device 15 comprises an outlet port 20 for connection to the pumping hose 25, for instance by means of a frictional connection that is established between the pumping hose 25 and the outlet port 20 of the adapter device 15 when the pumping hose 25 is plugged on the outlet port 20 of the adapter device 15, see figure 2.

Figures 1 to 4 depict the filter assembly 1 in a filtering state, wherein the filter holder 4 is partially received in the filter housing 6 and the filter membrane 5 is attached to the filter holder 4 to filter the sample from the fluid F. In fact, the jacket wall 9 and the filter membrane 5 are entirely received in the filter housing 6. However, the filter holder 4 and the filter membrane 5 are further configured for a storage state, wherein the filter holder 4 and the filter membrane 5 are at least partially insertable into a storage housing 28.

Namely, and as follows from figures 3, 4 and 5, the filter holder 4 is removably attached to the filter housing 6 such, that the filter holder 4 together with the filter membrane 5 is removable from the filter housing 6.

To this end, the filter housing 6 has a connection element 11 in the form of an external thread that can enter a connection with a connection element 12 in the form of an internal thread of the filter holder 4. In particular, the internal thread 11 is provided along an inner circumference of the lid 13. Upon insertion of the filter holder 4 into the filter housing 6 the filter holder 4 can be rotated with respect to the filter housing 6 about an axis of rotation R, whereby the lid 13 is screwed onto the filter housing 6. By an opposite rotary movement, the filter holder 4 together with the attached filter membrane 5 can be removed from the filter housing 6 and can be placed into the storage housing 28 of a storage assembly 100, see figures 5 and 6. Said state, wherein the filter holder, in particular the jacket wall 9, and the filter membrane 5 are inserted into the storage housing 28 is referred to as storage state. Hence, the filter holder 4 and the filter membrane 5 form part of both, the filter assembly 1 and the storage assembly 100.

As follows from figures 5 and 6, the storage housing 28 has an elongated, cylindrical shape and in particular the form of a storage tube. In the stored storage state, the storage housing 28, the filter membrane 5 and the filter holder 4 are arranged concentrically, see figure 6. In particular, in the storage state and when seen along the radial direction Rd of the filter holder 4 extending from the central axis C of the filter holder 4 radially outwards, the filter membrane 5 is arranged after the filter holder 4 and the storage housing 28 is arranged after the filter membrane 5. In other words, when seen in cross-section, it is preferred that the filter membrane 5 is arranged between the storage housing 28 and the filter holder 4.

The storage housing 28 comprises an opening 29 through which the filter holder 4 together with the filter membrane 5 is insertable into the storage housing 28. The storage housing 28 has a connection element 30 in the form of an external thread that is connectable with the connection element 12 in the form of the internal thread of the lid 13 of the filter holder 4 upon a rotation of the filter holder 4 relative to the storage housing 28 about a rotation axis R.

The storage assembly 100 further comprises a sealing element 16 in the form of a sealing cap that is configured to seal the outlet port 3 of the filter holder 4. Said sealing cap 16 has a connection element 17 in the form of an internal thread that can enter a connection with the connection element 18 in the form of the external thread of the outlet port 3, whereby the outlet port 3 of the filter holder 4 and thus the storage assembly 100 is sealed towards an outside.

This allows the filter membrane 5 with the filtered sample to be stored and, for instance, transported to a desired location, for example to a laboratory for analysis of the filtered sample.

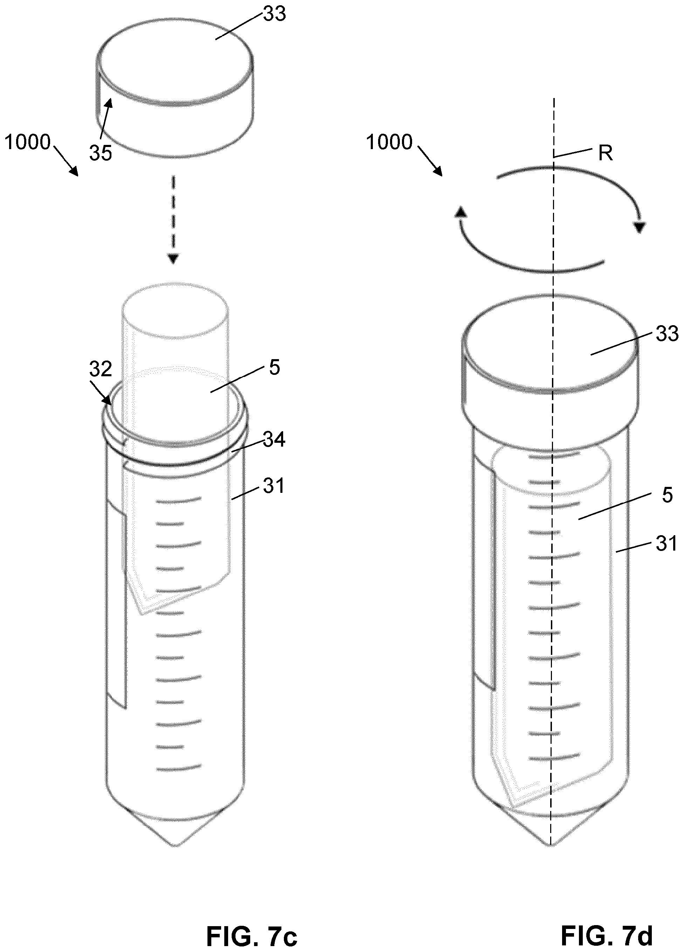

As can be seen from figures 7a and 7b, the filter holder 4 can be removed from the storage housing 28 together with the filter membrane 5 at a desired location, e.g. in a laboratory. Furthermore, the filter membrane 5 can also be removed from the filter holder 4, for example by stripping the filter membrane 5 from the jacket wall 9 of the filter holder 4, see figure 7b. The separate filter membrane 5 can then be placed in an analysis housing 31 of an analysis assembly 1000, see figures 7c and 7d. As follows from these figures, the analysis housing 31 can have an elongated, cylindrical shape and could in turn be a centrifuge tube with an opening 32 for inserting the filter membrane 5. After insertion of the filter membrane 5, the opening 32 can be closed with a closure element 33 such as a screw cap, which has a connection element 35 such as an internal thread that enters a connection with a connection element 34 such as an external thread of the analysis housing 31 upon a rotation of the closure element 33 with respect to the analysis housing 31 about an axis of rotation 3. In this state, depicted in figure 7d, the filter membrane 5 together with the analysis assembly 1000 can be subjected to an analysis device being configured to analyse the filtered sample, for example.

### LIST OF REFERENCE SIGNS

- 1: filter assembly
- 2: inlet port
- 3: outlet port
- 4: filter holder
- 5: filter membrane
- 6: filter housing
- 7: outer surface of filter holder
- 8: interior space
- 9: jacket wall
- 10: through-opening of filter holder
- 11: connection element of filter housing
- 12: connection element of filter holder
- 13: lid
- 14: opening
- 15: adapter device
- 16: sealing element
- 17: connection element of sealing element
- 18: connection element of outlet port
- 19: pumping device
- 20: outlet port of adapter device
- 21: bucket
- 22: lower side
- 23: upper side
- 24: inner surface filter membrane
- 25: pumping hose
- 26: connection element of adapter device
- 27: through-opening
- 28: storage housing
- 29: opening
- 30: connection element of storage housing
- 31: analysis housing
- 32: opening
- 33: closure element
- 34: connection element
- 35: connection element

- 100: storage assembly
- 1000: analysis assembly

- F: fluid
- R: rotation axis
- C: central axis of filter holder
- C1: central axis of filter assembly
- Rd: radial direction of filter holder
- L: longitudinal direction of filter holder

## Claims

1. A filter assembly (1) for filtering a sample from a fluid (F) comprising:
- an inlet port (2) and an outlet port (3),
- a filter holder (4),
- a filter membrane (5), and
- a filter housing (6),
wherein the filter holder (4) is at least partially received in the filter housing (6),
wherein the filter membrane (5) is attached to the filter holder (4) and is configured to filter a sample from a fluid (F) flowing from the inlet port (2) to the outlet port (3) through the filter membrane (5),
**characterized in that** the filter holder (4) is removably attached to the filter housing (6) such, that the filter holder (4) together with the filter membrane (5) is removable from the filter housing (6).

2. The filter assembly (1) according to claim 1, wherein the filter holder (4) together with the filter membrane (5) is indestructibly removable from the filter housing (6), and/or
wherein the filter holder (4) and/or the filter housing (6) are at least one of: re-usable, UV-resistant, or bleach resistant.

3. The filter assembly (1) according to any one of the preceding claims, wherein the filter membrane (5) is attached to an outer surface (7) of the filter holder (4) and preferably extends at least partially around the outer surface (7) of the filter holder (4), and/or
wherein the filter membrane (5) is removably attached to the filter holder (4), preferably via a frictional fit.

4. The filter assembly (1) according to any one of the preceding claims, wherein the filter housing (6) and the filter holder (4) in each case have at least one connection element (11; 12) that can be detachably connected to each other, the connection elements (11; 12) preferably being threaded elements, and/or
wherein the filter holder (4) is rotatably mounted to the filter housing (6) and connectable to the filter housing (6) via rotation about an axis of rotation (R).

5. The filter assembly (1) according to any one of the preceding claims, wherein the filter holder (4) comprises a lid (13) and the filter housing (6) comprises an opening (14), wherein the filter holder (4) is insertable into the filter housing (6) via the opening (14) of the filter housing (6), and wherein the lid (13) of the filter holder (4) is configured to at least partially close the opening (14) of the filter housing (6) when the filter holder (4) is received in the filter housing (6), and/or
wherein the filter holder (4), preferably the lid (13), comprises the outlet port (3), and/or
wherein the filter housing (6) comprises the inlet port (2).

6. The filter assembly (1) according to any one of the preceding claims, wherein the outlet port (3) is connectable to a pumping device (19) being configured to generate a suction force in the filter assembly (1) that sucks the fluid (F) into the filter assembly (1) through the inlet port (2) and out of the filter assembly (1) through the outlet port (3), the pumping device (9) preferably being a hand-operated pumping device, and/or
wherein the filter assembly (1) further comprises at least one adapter device (15) that is connectable to the outlet port (3) and further connectable to a pumping device (19), preferably via at least one pumping hose.

7. A storage assembly (100) comprising:
- at least one storage housing (28), and
- at least one filter holder (4) and filter membrane (5) according to any one of the preceding claims,
wherein the filter holder (4) together with the filter membrane (5) can be stored in the storage housing (28).

8. The storage assembly (100) according to claim 7, wherein the storage assembly (100) comprises at least one sealing element (16), preferably a sealing cap, that is configured to seal the outlet port (3) of the filter holder (4), and
wherein the sealing element (16) and the outlet port (3) preferably in each case have at least one connection element (17; 18) that can enter a preferably releasable connection with one another.

9. A kit-of-parts for filtering a sample from a fluid (F) and for storing the filtered sample comprising:
- at least one filter assembly (1) as claimed in any one of claims 1 to 6, and
- at least one storage housing (28) as claimed in claim 7 or 8,
wherein the filter holder (4) together with the filter membrane (5) can be stored in the storage housing (28).

10. The kit-of-parts as claimed in claim 9, wherein the kit-of-parts comprises instructions comprising the steps of:
- filtering the sample from the fluid (F) with the filter assembly (1),
- removing the filter holder (4) together with the filter membrane (5) from the filter housing (6) of the filter assembly (1), and
- transferring the filter holder (4) together with the filter membrane (5) into the storage housing (28).

11. The kit-of-parts according to claim 9 or 10, wherein the storage housing (28) is prefilled with a preservation solution that is configured to preserve a sample being filtered with the filter assembly (1).

12. The kit-of-parts as claimed in any one of claims 9 to 11, wherein the kit-of-parts comprises at least one pumping device (19) that is connectable to the outlet port (3) and is configured to generate a suction force in the filter assembly (1) that sucks the fluid into the filter assembly (1) through the inlet port (2) and out of the filter assembly (1) through the outlet port (3), the pumping device (19) preferably being a hand-operated pumping device, and/or
wherein the kit-of-parts comprises at least one adapter device (15) that is connectable to the outlet port (3) and further connectable to a pumping device (19), preferably via at least one pumping hose, and/or
wherein the kit-of-parts comprise at least one analysis assembly (1000) that is configured to at least partially receive the filter membrane (5) and to be analysed with an analysis device being configured to analyse the filtered sample.

13. A method of filtering a sample from a fluid (F) and of storing the filtered sample comprising the steps of:
- providing a kit-of-parts as claimed in any one of claims 9 to 12;
- filtering the sample from the fluid (F) with the filter assembly (1),
- removing the filter holder (4) together with the filter membrane (5) from the filter housing (6) of the filter assembly (1), and
- transferring the filter holder (4) together with the filter membrane (5) into the storage housing (28),
wherein the sample preferably is environmental DNA and the fluid (F) preferably is an environmental fluid such as environmental water or an environmental gas.

14. A method of analyzing a sample from a fluid comprising the steps of:
- filtering a sample from a fluid (F) and storing the filtered sample with a kit-of-parts as claimed in any one of claims 9 to 12 and/or according to the method of claim 13;
- removing the filter holder (4) together with the filter membrane (5) from the storage housing (28); and
- analyzing the filter membrane (5) comprising the filtered sample preferably after the filter membrane (5) has been removed from the filter holder (4) and optionally has been transferred into the analysis assembly (1000),
wherein the sample preferably is environmental DNA and the fluid (F) preferably is an environmental fluid such as environmental water or an environmental gas.

15. A method of manufacturing a kit-of-parts comprising the steps of:
- Providing at least one filter assembly (1) as claimed in any one of claims 1 to 6, and
- Providing at least one storage housing (28) as claimed in claim7 or 8,
wherein at least one of: the filter holder (4), the filter housing (6), and the storage housing (28) are preferably provided by 3D-printing or molding.
